Europäisches Patentamt

European Patent Office

Office européen des brevets

(11) Veröffentlichungsnummer : **0 057 281
B1**

(12) **EUROPÄISCHE PATENTSCHRIFT**

(45) Veröffentlichungstag der Patentschrift :
**17.10.84**

(21) Anmeldenummer : **81110396.9**

(22) Anmeldetag : **12.12.81**

(51) Int. Cl.³ : **C 07 C 69/732, C 07 C 67/36**

(54) **Verfahren zur Herstellung von alpha-Hydroximethylenarylessigestern.**

(30) Priorität : **31.01.81 DE 3103308**

(43) Veröffentlichungstag der Anmeldung :
**11.08.82 Patentblatt 82/32**

(45) Bekanntmachung des Hinweises auf die Patenterteilung : **17.10.84 Patentblatt 84/42**

(84) Benannte Vertragsstaaten :
**AT CH DE FR GB IT LI NL**

(56) Entgegenhaltungen :
**Keine**

(73) Patentinhaber : **DYNAMIT NOBEL AKTIENGESELL-
SCHAFT
Postfach 1209
D-5210 Troisdorf, Bez. Köln (DE)**

(72) Erfinder : **Englaender, Fritz, Dr.
Flossweg 10
D-5300 Bonn 2 (DE)**
Erfinder : **El-Chahawi, Moustafa, Dr.
Pacellistrasse 4
D-5210 Troisdorf (DE)**
Erfinder : **Vogt, Wilhelm, Dr.
Kleiststrasse 39
D-5000 Köln 40 (DE)**

EP 0 057 281 B1

## Beschreibung

Die Erfindung betrifft ein verbessertes Verfahren zur Herstellung von α-Hydroximethylenarylessigsäurealkylestern aus den entsprechenden Arylessigestern unter Verwendung von Alkalialkoholat und Kohlenoxid bei erhöhter Temperatur und Druck.

Es ist bekannt, daß Phenylessigsäuremethylester mit metallischem Natrium oder Kalium und Ameisensäuremethylester in Aether als Lösungsmittel das Natriumsalz des α-Hydroximethylenphenylessigsäuremethylesters bildet (Wislicenus, Annalen der Chemie : 282 (1984) 231). Die Reaktion ist unvollständig und die Abtrennung von nicht umgesetzten Phenylessigsäureester schwierig.

Zur technischen Herstellung von α-Hydroximethylenphenylessigsäuremethylester ist dieses Verfahren wegen der Verwendung metallischen Natriums wenig geeignet.

Es wurde nun gefunden, daß hohe Ausbeuten und Reinheiten von α-Hydroximethylenarylessigsäurealkylestern durch Umsetzung von Arylessigsäurealkylestern mit Natriumalkoholat und CO bei erhöhter Temperatur und erhöhtem Druck erhalten werden.

Im Gegensatz zu der gefährlichen und umständlichen Arbeitsweise mit Alkalimetallen ist das Verfahren einfach und gefahrlos sowie mit sehr hohen Ausbeuten bis zu 95 % der Theorie ausführbar.

Die Reaktionszeit beträgt nach dem erfindungsgemäßen Verfahren etwa 3 Stunden, während die Umsetzung von Ameisensäureester mit Alkalimetall 24 Stunden dauert.

Gegenstand der Erfindung ist ein Verfahren zur Herstellung von α-Hydroximethylenarylessigsäurealkylestern, dadurch gekennzeichnet, daß man Arylessigsäurealkylester der allgemeinen Formel $R_1CH_2CO_2R_2$, in welcher $R_1$ einen Arylrest mit einem oder zwei aromatischen Ringen und $R_2$ einen geradkettigen oder verzweigten Alkylrest bedeutet, in Gegenwart eines Alkalialkoholats aliphatischer Alkohole mit 1 bis 6 C-Atomen mit CO bei Drucken von 20 bis 200 bar und bei Temperaturen von 30 bis 150 °C umsetzt.

Bevorzugt sind Temperaturen von 40 bis 85 °C und ein CO-Druck von 80 bis 100 bar.

Umsatz und Ausbeute ist praktisch quantitativ. Sehr bemerkenswert ist die hohe Reinheit der Produkte.

Nach beendeter Umsetzung erhält man eine Suspension des Alkalisalzes der α-Hydroximethylenarylessigsäurealkylester in dem verwendeten Lösungsmittel. Durch Zugabe dieser Suspension in überschüssige wässerige Säure, bevorzugt Salzsäure oder Schwefelsäure bildet sich die freie α-Hydroximethylenverbindung, welche sich in dem Lösemittel löst, während das entstandene NaCl mit dem Wasser abgetrennt wird. Aus der organischen Phase kann der α-Hydroximethylenarylessigsäurealkylester leicht durch Destillation oder Kristallisation gewonnen werden. Als Lösemittel können solche mit Siedepunkten von 35 bis 180 °C eingesetzt werden, wie aliphatische und aromatische Kohlenwasserstoffe, wie die Benzinfraktionen Benzol, Toluol oder die Xylole, aliphatische Äther wie 2 bis 6 C-Atomen oder Dioxan, aliphatische Alkohole mit 1 bis 4 C-Atomen.

Es ist auch möglich, den Ausgangsstoff als Lösemittel einzusetzen. Bevorzugte Lösemittel sind aromatische Kohlenwasserstoffe, besonders Toluol. Art und Menge des Lösemittels wird bevorzugt so gewählt, daß aus der entstehenden Suspension des Produkts kein Feststoff ausfällt.

Als Arylessigester werden insbesondere solche mit Alkoholresten $R_2$ von 1 bis 6 C-Atomen, besonders die Methyl- und Äthylester eingesetzt ; Der Arylrest $R_1$ der Ester kann einen oder zwei aromatische Ringe enthalten, wobei die aromatischen Ringe entweder unsubstituiert sind, d. h. nur Wasserstoff als Substituent enthalten, oder einen oder mehrere bei der Reaktion inerte Substituenten, besonders Halogene wie Chlor oder Brom, niedere Alkylreste von 1 bis 4 C-Atomen, bevorzugt den Methylrest oder Ethylrest enthalten.

Bevorzugt sind substituierte oder unsubstituierte Phenylreste, α- bzw. β-Naphthylreste, Biphenylreste.

Das verwendete Alkalialkoholat ist bevorzugt Natriummethylat und Natriumäthylat. Es können jedoch auch Alkoholate anderer aliphatischer Alkohole mit 1 bis 6 C-Atomen eingesetzt werden. Im allgemeinen wird zur Vermeidung von Nebenreaktionen das Alkoholat verwendet, dessen Alkoholrest dem des Esters entspricht, z. B. bei einem Methylester Natriummethylat. Sofern Alkohole als Lösemittel Verwendung finden, ist es Ethanol für Ethylester als Ausgangsstoff und Methanol für Methylester.

Obwohl die Natriumalkoholate sehr bevorzugt sind, können auch Alkoholate des Kaliums oder gegebenenfalls Lithiums benutzt werden. Das Alkoholat kann als feste Substanz, gelöst im jeweiligen Lösungsmittel, bevorzugt in Toluol oder gegebenenfalls als Lösung im entsprechenden Alkohol verwendet werden.

Bei der Reaktion wird bevorzugt ein Überschuß des Alkoholats gegenüber der Ester angewandt ; außer in den Fällen, in denen der Ester als Lösemittel dient. Der Überschuß des Alkoholats kann 0,5 bis 20, bevorzugt 2,5 bis 5 Mol-%, betragen.

Die α-Hydroximethylenarylessigsäurealkylester sind vielseitig verwendbare Zwischenprodukte zur Herstellung heterocyclischer Verbindungen z. B. von 7-Aminocumarinen nach der DE-AS 12 78 444.

Beispiel 1

150 Gewichtsteile Phenylessigsäuremethylester, 56,7 Gewichtsteile Natriummethylat und 516 Gewichtsteile Toluol werden bei einem CO-Druck von 100 bar auf 55 °C aufgeheizt. Ab einer Temperatur von 45 °C findet eine rasche CO-Aufnahme statt. Das verbrauchte CO wird durch frisches ersetzt, so daß der Druck zwischen 90 und 100 bar schwankt. Sobald kein Druckabfall mehr eintritt, wird abgekühlt und das CO abgeblasen. Die Suspension wird in 300 Gewichtsteile 12,8 Gew.-%ige wässrige Salzsäure gegeben und so lange gerührt, bis das Na-Salz in Lösung gegangen ist. Die Toluolphase wird abgetrennt und destilliert. Man erhält 166 Gewichtsteile (entsprechend 93,3 % d. Th.) an α-Hydroximethylenphenylessigsäuremethylester mit einem Schmelzpunkt von 41 °C.

Nach der gaschromatographischen Analyse besaß die Verbindung eine Reinheit von 99,9 %.

Beispiel 2

Unter den gleichen Reaktionsbedingungen wie bei Beispiel 1 wurden a) 4-Chlorphenylessigsäuremethylester und b) 2-Chlorphenylessigsäuremethylester umgesetzt. Nach der Aufarbeitung erhält man

a) α-Hydroximethylen-4-chlorphenylessigsäuremethylester in einer Ausbeute von 88,2 % bzw.

b) α-Hydroximethylen-2-chlorphenylessigsäuremethylester in einer Ausbeute von 84,8 %.

Cl ber. : 16,67 %
Cl gef. : 16,71 %     α-Hydroximethylen-4-chlorphenylessigsäuremethylester
Cl gef. : 16,83 %     α-Hydroximethylen-2-chlorphenylessigsäuremethylester

Beispiel 3

174 Gewichtsteile α-Naphthylessigsäuremethylester, 48,1 Gewichtsteile Natriummethylat und 450 Gewichtsteile Toluol werden bei einem CO-Druck von 100 bar auf 80 °C geheizt. Das verbrauchte CO wird durch frisches CO ersetzt, so daß der Druck zwischen 90 und 100 bar schwankt. Nach Beendigung der Reaktion wird abgekühlt und das nicht reagierte CO abgeblasen. Die Suspension des Na-Salzes in Toluol wird in I-Ltr.-Eiswasser gegeben und nach Auflösung des Na-Salzes die Toluolphase abgetrennt.

Beim Ansäuern der wässrigen Phase erhält man den α-Hydroximethylen-α-naphthylessigsäuremethylester in einer Reinheit von 99,5 % (gaschromatographisch) und einen Schmelzpunkt von 124-126 °C.

|     | ber. | gef. |
|-----|------|------|
| C : | 73,67 | 73,92 |
| H : | 5,3 | 5,66 |

**Ansprüche**

1. Verfahren zur Herstellung von α-Hydroximethylenarylessigsäurealkylestern, dadurch gekennzeichnet, daß man Arylessigsäurealkylester der allgemeinen Formel $R_1CH_2CO_2R_2$, in welcher $R_1$ einen Arylrest mit einem oder zwei aromatischen Ringen und $R_2$ einen geradkettigen oder verzweigten Alkylrest bedeutet, in Gegenwart eines Alkalialkoholats aliphatischer Alkohole mit 1 bis 6 C-Atomen mit CO bei Drucken von 20 bis 200 bar und bei Temperaturen von 30 bis 150 °C umsetzt.

2. Verfahren nach Anspruch 1, dadurch gekennzeichnet, daß die Reaktion in Gegenwart eines Lösungsmittels ausgeführt wird.

3. Verfahren nach mindestens einem der Ansprüche 1 oder 2, dadurch gekennzeichnet, daß nach beendeter Reaktion aus dem Na-Salz des α-Hydroximethylenarylessigsäurealkylesters durch Umsetzung mit einer Säure der α-Hydroximethylenarylessigsäurelakylester in Freiheit gesetzt wird.

**Claims**

1. Process for the preparation of α-hydroxymethylenearylacetic acid alkyl esters, characterised in that arylacetic acid alkyl esters of the general formula $R_1CH_2CO_2R_2$, in which $R_1$ denotes an aryl residue with one or two aromatic rings and $R_2$ a straight-chained or branched alkyl residue is reacted, in the presence of an alkali alcoholate of aliphatic alcohol with 1 to 6 C-atoms, with CO at pressures of 20 to 200 bar and at temperatures of 30 to 150 °C.

2. Process according to claim 1, characterised in that the reaction is carried out in the presence of a solvent.

3. Process according to at least one of claims 1 or 2, characterised in that, after the end of the reaction, the α-hydroxymethylenearylacetic acid alkyl ester is liberated from the Na-salt of the α-hydroxymethylenearylacetic acid alkyl ester by reaction with an acid.

**Revendications**

1. Procédé pour la préparation d'α-hydroxyméthylènearylacétates d'alcoyle, caractérisé en ce que l'on fait réagir des arylacétates d'alcoyle de formule générale $R_1CH_2CO_2R_2$ dans laquelle $R_1$ représente un résidu aryle avec un ou deux cycles aromatiques et $R_2$ un résidu alcoyle à chaîne droite ou ramifiée, avec du CO sous des pressions de 20 à 200 bars et à des températures de 30 à 150 °C en présence d'un alcoolate alcalin d'alcools aliphatiques renfermant 1 à 6 atomes de carbone.

2. Procédé selon la revendication 1, caractérisé en ce que la réaction est conduite en présence d'un solvant.

3. Procédé selon au moins une des revendications 1 ou 2, caractérisé en ce que, après la fin de la réaction, l'α-hydroxyméthylènearylacétate d'alcoyle est libéré à partir du sel de Na de l'α-hydroxyméthylènearylacétate d'alcoyle par réaction avec un acide.